(19) **Europäisches Patentamt**
**European Patent Office**
**Office européen des brevets**

(11) **EP 2 036 044 B1**

(12) **FASCICULE DE BREVET EUROPEEN**

(45) Date de publication et mention
de la délivrance du brevet:
**08.08.2012 Bulletin 2012/32**

(51) Int Cl.:
*G06T 7/00* *(2006.01)*          *A61B 5/055* *(2006.01)*

(86) Numéro de dépôt international:
**PCT/FR2007/001111**

(21) Numéro de dépôt: **07803820.5**

(22) Date de dépôt: **29.06.2007**

(87) Numéro de publication internationale:
**WO 2008/000973 (03.01.2008 Gazette 2008/01)**

(54) **MÉTHODE D'ESTIMATION DU POTENTIEL DE CROISSANCE DES INFARCTUS CÉRÉBRAUX**

VERFAHREN ZUR SCHÄTZUNG DES WACHSTUMSPOTENZIALS ZEREBRALER INFARKTE

METHOD FOR ESTIMATING THE GROWTH POTENTIAL OF CEREBRAL INFARCTS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **29.06.2006 US 817467 P**

(43) Date de publication de la demande:
**18.03.2009 Bulletin 2009/12**

(73) Titulaire: **INTELLIGENCE IN MEDICAL TECHNOLOGIES**
**75002 Paris (FR)**

(72) Inventeurs:
- **BAILLET, Sylvain**
  **F-78140 Velizy-Villacoublay (FR)**
- **SAMSON, Yves**
  **F-75010 Paris (FR)**
- **HEVIA-MONTIEL, Nidiyare**
  **Morelos C.P. 62130 (MX)**
- **ROSSO, Charlotte**
  **F-75015 Paris (FR)**
- **DELTOUR, Sandrine**
  **F-94300 Vincennes (FR)**
- **BARDINET, Eric**
  **F-91190 Gif Sur Yvette (FR)**
- **DORMONT, Didier**
  **F-94300 Vincennes (FR)**

(74) Mandataire: **Novagraaf Technologies**
**122 rue Edouard Vaillant**
**92593 Levallois-Perret Cedex (FR)**

(56) Documents cités:
**WO-A-02/069799      WO-A-2007/058632**

- **GOSSRAU ET AL: "Wertigkeit des Apparent Diffusion Coefficient (ADC) in der Funktionsdiagnostik der grossen Speicheldr} sen und der Beurteilung pathologischer Veraenderungen mittels Magnetresonanztomographie bei 1,5T und 3T (Dissertation: universitaet Hamburg, FB Medizin)" INTERNET CITATION, [Online] 2005, XP007903763 Extrait de l'Internet: URL:http: //www.sub.uni-hamburg.de/opus/vol ltexte/ 2005/2632/pdf/online_diss.p> [extrait le 2008-01-03]**
- **LI ET AL: "Temporal evolution of average apparent diffusion coefficient threshold to define ischemic abnormalities in a rat permanent occlusion model" JOURNAL OF STROKE AND CEREBROVASCULAR DISEASES, DEMOS PUBLICATIONS, NEW YORK, NY, US, vol. 9, no. 1, février 2000 (2000-02), pages 1-7, XP022292157 ISSN: 1052-3057**
- **BURDETTE J H ET AL: "Cerebral infarction: time course of signal intensity changes on diffusion-weighted MR images." AJR. AMERICAN JOURNAL OF ROENTGENOLOGY SEP 1998, vol. 171, no. 3, septembre 1998 (1998-09), pages 791-795, XP007903768 ISSN: 0361-803X**

## Description

L'invention concerne une méthode d'estimation automatique du potentiel de croissance des infarctus cérébraux en particulier en phase aiguë, c'est-à-dire dans les six heures suivant la survenue de l'accident ischémique cérébral.

À ce titre, l'invention concerne le domaine de l'imagerie cérébrale et plus particulièrement l'analyse et le traitement d'images obtenues par résonance magnétique (IRM) pour déterminer le potentiel de croissance des infarctus cérébraux durant leur phase aiguë. L'intérêt de cette démarche est de déterminer de façon précoce le rapport bénéfices/risques que présente la mise en oeuvre de traitements, efficaces mais agressifs, pour lutter contre la propagation de ces accidents vasculaires cérébraux.

Il est connu de l'état de la technique quelques méthodes utilisées sous forme d'outils logiciels non encore standardisés. Les méthodes actuelles de prédiction de croissance des infarctus cérébraux à partir de données d'imagerie sont essentiellement basées sur la comparaison des anomalies des séquences IRM de perfusion et de diffusion, ou sur l'étude des images issues du scanner de perfusion.

À ce titre, la demande de brevet internationale n° WO 07/058632 décrit les différentes étapes permettant d'obtenir une estimation du potentiel de croissance de l'infarctus, selon de telles méthodes. Malgré leur intérêt théorique majeur, toutes ces méthodes se sont heurtées à des limitations physiopathologiques et méthodologiques diverses. Leur standardisation s'est révélée extrêmement complexe et aucune d'entre elles ne s'est imposée comme un standard incontournable. De ce fait, leur mise en oeuvre reste confinée au cadre de la recherche physiopathologique ou éventuellement à celui d'essais thérapeutiques à grande échelle auxquels ne peuvent participer que quelques centres hautement spécialisés.

Les seules approches actuelles dont fait état la littérature, consistent à évaluer une disparité entre deux types d'images IRM acquises séquentiellement chez le patient : une image de diffusion qui permet d'évaluer l'étendue de l'infarctus déjà établi et une image de perfusion qui donne un indice de la pénombre ischémique, c'est-à-dire de l'étendue du tissu neuronal en souffrance mais pouvant encore être sauvé par intervention thérapeutique. Plus la disparité de volume entre les régions identifiées dans chaque série d'images est grande, plus le risque de croissance est considéré comme important.

Cette méthode, *a priori* simple et pertinente, est en fait assez complexe à mettre en oeuvre. L'injection nécessaire d'un produit de contraste (par exemple le gadolinium) allonge l'examen lors de la séquence de perfusion, notamment lorsque l'accès veineux est difficile, ce qui n'est pas adapté à un contexte clinique d'urgence. Par ailleurs, les méthodes de quantification de mesures de perfusion restent également encore débattues et peu standardisées et leurs propriétés de reproductibilité ne sont pas satisfaisantes. Enfin et surtout, aucune méthode n'a permis d'obtenir, un ratio sensibilité/spécificité satisfaisant avec un temps de traitement d'image compatible avec une utilisation d'urgence. L'analyse de la littérature suggère que si la sensibilité de cette méthode est correcte (70 à 80%), ses performances en termes de spécificité de détection de croissance sont médiocres.

La demande de brevet internationale n° WO 02/069799 décrit une méthode d'estimation de l'évolution des infarctus cérébraux à partir d'une séquence d'images IRM de diffusion et de perfusion qui est basée sur un algorithme de classification.

Le but de l'invention est de proposer une méthode pouvant être mise en oeuvre de façon standardisée et permettant de prédire de façon automatique, rapide et fiable, le potentiel de croissance d'un infarctus cérébral chez un patient venant de subir un accident ischémique cérébral.

Cette méthode permet de fournir un outil d'aide à la décision thérapeutique d'extrême urgence à l'échelle individuelle, ou d'évaluation rapide de nouveaux traitements pour l'industrie pharmaceutique sur un petit groupe de patients. Dans ce dernier cas, l'effet d'une molécule en phase de test peut-être évalué rapidement par comparaison entre la croissance effective de l'infarctus et celle prédite par la méthode d'estimation selon l'invention. Si la molécule est efficace à réduire la croissance de l'infarctus, la méthode d'estimation selon l'invention fournira systématiquement une surestimation de cette dernière.

L'invention exploite le fait que, lors de la phase aiguë de l'infarctus, la valeur du coefficient de diffusion apparent (CDA) est réduite de façon importante dans les régions déjà infarcies, mais aussi, de façon plus modérée, dans la zone de pénombre ischémique, c'est-à-dire la zone susceptible d'être définitivement infarcie dans les heures qui suivent. En détectant de manière automatique la région entourant le coeur de l'infarctus dont le CDA moyen est plus faible que la normale, il est possible d'accéder à une estimation de la taille finale de l'infarctus. L'évaluation des risques vitaux ou fonctionnels encourus par le patient est alors plus complète.

Si la valeur régionale du CDA est centrale dans cette méthode, d'autres critères sont également pris en compte afin de modéliser au mieux la croissance de la lésion. Ces critères supplémentaires portent sur :

- le choix des éléments (voxels) de l'image de IRM candidats à l'inclusion dans le modèle d'infarctus en croissance ;
- la régularité de la forme globale de la lésion ;
- la direction de la croissance dans le volume du parenchyme cérébral en fonction des propriétés d'anisotropie des mesures CDA ;

- l'adéquation à un atlas probabiliste numérique de l'étendue des régions lésées pour le type d'occlusion initiale concernée.

Plus précisément, l'invention a pour objet une méthode d'estimation du potentiel de croissance des infarctus cérébraux, la méthode comportant les étapes suivantes : acquisition de séquences d'images IRM de diffusion, calcul du coefficient de diffusion apparent (CDA) en une multitude de points ou voxels du parenchyme cortical, localisation et délimitation de l'infarctus initial et modélisation de l'évolution de l'infarctus à partir d'un modèle de croissance établie par la minimisation itérative d'un indice d'énergie globale E définie par une combinaison linéaire de paramètres d'énergie élémentaire $E_1$ dépendant de l'intensité du CDA.

Cette méthode d'estimation du potentiel de croissance des infarctus cérébraux selon l'invention ne nécessite qu'une séquence d'images IRM pondérées en diffusion sur un imageur IRM standard et couramment à disposition dans les services de neuroradiologie. La méthode ne nécessite donc pas l'admission par voie intraveineuse d'un agent de contraste et le temps d'acquisition des images IRM est réduit à moins de 5 minutes.

D'autre part, cette méthode est très simple à mettre en oeuvre puisque l'analyse des cartes en 3D de la mesure du CDA dans le volume du parenchyme cérébral ne dépend absolument pas de l'imageur utilisé.

Enfin la valeur régionale moyenne du CDA au sein de la région atteinte par l'infarctus final a déjà été explorée dans la littérature scientifique.

Avantageusement, la combinaison linéaire comporte les paramètres d'énergie élémentaires $E_1$ suivants :

- $E_R$ selon lequel la valeur régionale moyenne du CDA à l'intérieur de la région d'infarctus en croissance tend vers une valeur cible pré-établie ;
- $E_S$ selon lequel l'enveloppe de l'infarctus possède une surface régulière ;
- $E_V$ selon lequel seuls les voxels modélisant le tissu cérébral sont pris en compte dans le calcul de cet indice d'énergie globale ;
- $E_P$ définissant les probabilités de croissance de l'infarctus à chaque voxel à partir de schémas empiriques ;
- $E_{AN}$ définissant un modèle de croissance anisotrope, réalisé par le calcul du gradient local du coefficient de diffusion apparent.

De préférence, ces paramètres d'énergie élémentaires $E_R$, $E_S$, $E_V$, $E_P$ et $E_{AN}$ se traduisent par les fonctions mathématiques suivantes :

$$E_R = \left( \frac{i_{INF} - \bar{\bar{i}}_{INF}}{\sigma_{INF}} \right)^2,$$

où

- $i_{INF}$ (resp. $\sigma_{INF}$) est la moyenne empirique (resp. l'écart type) de l'intensité du CDA dans la région en croissance *INF ;*
- $\bar{i}_{INF}$ est une valeur cible de la moyenne régionale coeur de la zone de pénombre ischémique.

$$E_S = \sum_{r \in INF} \left( \frac{N_v - N/2}{\delta} \right)^{\beta},$$

où
- $N_{INF}$ (resp. $N_{IG}$) est le nombre de voxels dans les régions *INF* et *IG*, respectivement ;
- $E_S$ est potentiel de régularisation selon Ising, bien connu de la communauté en traitement numérique des images ;
- $N$ est le nombre total de voxels voisins à un voxel candidat à l'inclusion dans la région *INF* (26 par exemple, en 3 dimensions), dont $N_v$ appartiennent déjà *INF* ;
- $\delta$ et $\beta$ sont des paramètres scalaires fixes.

$$E_V = \sum_{v \in IG} \left( \frac{i(v) - \bar{i}_{IG}}{\sigma_{IG}} \right)^2,$$

où
- $i(v)$ est la valeur du CDA au voxel $v$ ;
- $\bar{i}_{IG}$ (resp. $\sigma_{IG}$) est un a priori concernant les valeurs de CDA moyennes (resp. en écart type) aux voxels dans la région de croissance $IG$ ;
- $N_{INF}$ (resp. $N_{IG}$) le nombre de voxels dans les régions $INF$ et $IG$, respectivement.

$$E_p = \sum_{v \in INF} p(v),$$

où
- p(v) est la probabilité a priori que le voxel v appartiennent à l'infarctus dans sa forme finale.

$$E_{AN} = \frac{1}{\left\| \vec{V}_{CDA} \right\| \left\| \vec{V}_{INF} \right\|} \sum_{v \in INF} \vec{V}_{CDA}(v) \cdot \vec{V}_{INF}(v),$$

où
- $\vec{V}_{CDA}$ est le flot de vecteurs gradients définit en chaque voxel de la carte de CDA ; et
- $\vec{V}_{INF}$ les gradients calculés en tout point du masque binaire de la lésion en croissance.

La minimisation énergétique de l'état de la lésion en tant qu'objet virtuel permet d'introduire plusieurs paramètres indépendants et d'affiner la sensibilité des résultats obtenus.

Selon des caractéristiques particulières, la valeur cible pré-établie vers laquelle tend la valeur régionale moyenne du CDA à l'intérieur de la région en croissance est sensiblement égale à 740 mm$^2$.s$^{-1}$ ou 0.93 fois la valeur moyenne du CDA dans une région saine controlatérale.
Cette valeur régionale moyenne du CDA au sein de l'infarctus final a déjà été explorée dans la littérature scientifique et est sensiblement identique d'un patient à l'autre. Par conséquent, aucun réglage n'est nécessaire pour obtenir de bons résultats.

Le paramètre $E_S$ permet d'éviter toute aberration topologique incompatible avec la neurophysiopathologie de la lésion ischémique en croissance.

Le paramètre $E_V$ permet d'éviter les aberrations concernant la valeur du CDA en chaque voxel. Si cette dernière est trop faible, il s'agit éventuellement d'un voxel à la valeur de CDA bruitée ou artefactée ; si elle est trop élevée, il s'agit éventuellement d'un voxel du liquide céphalo-rachidien, indésirable dans la lésion.

Le paramètre $E_P$ guide la croissance de l'infarctus par une carte de probabilité empirique d'atteinte de chaque voxel de l'image par la lésion. Cette carte peut être disponible pour chaque type d'occlusion originelle ayant menée à l'infarctus ischémique.

Enfin le paramètre $E_{AN}$ contrôle la direction préférentielle de croissance de la lésion en 3D, en fonction de l'anisotropie de la distribution du CDA en chaque point de la surface de la lésion en croissance. L'infarctus croîtra préférentiellement dans la direction de moindre gradient d'intensité de CDA local. L'estimation de l'infarctus final est donc plus fiable.

L'invention concerne également un dispositif permettant la mise en oeuvre d'une méthode d'estimation du potentiel de croissance selon l'invention. Ainsi, une fois initialisé par un masque de la lésion en phase aiguë, ce dispositif permet d'obtenir simplement, rapidement et de façon automatique, un avis fiable sur la croissance de la lésion.

D'autres caractéristiques et avantages de l'invention ressortiront à la lecture qui suit d'exemples de réalisation détaillés, en référence aux figures qui représentent respectivement :

- La figure 1, un schéma symbolique du dispositif permettant la mise en oeuvre d'une méthode d'estimation du potentiel de croissance selon l'invention ;

- La figure 2, un organigramme séquentiel permettant la mise en oeuvre d'une méthode d'estimation du potentiel de croissance des infarctus cérébraux selon l'invention ; et
- la figure 3, une séquence d'images numériques représentant les croissances réelles et prédites de l'infarctus cérébral chez un patient.

Le terme « image » employé dans la description qui suit fait référence aux données rendant compte de la nature du tissu cérébral d'un patient en de multiples points de l'espace. Ces « images » sont donc constituées d'une multitude de points permettant de représenter un espace en deux ou trois dimensions.

Les « points » numérisés formant l'image désignent des voxels (*volumetric pixel*) ou des pixels selon que l'image est issue ou non d'une série de coupes explorant une partie de l'espace en trois dimensions.

Le terme « carte » fait référence à des images, en deux ou trois dimensions, représentant la répartition spatiale de certaines propriétés des tissus, également appelé « parenchyme », constituant la tête. Ces cartes peuvent provenir soit de bases de données afin de servir de modèles adaptables aux spécificités de chaque patient, soit de l'exploitation des données individuelles recueillies lors de l'acquisition d'images sur un patient. Ces cartes renseignent sur la structure du cerveau ou bien sur l'état du tissu cérébral d'un patient. Avantageusement, les cartes peuvent se superposer afin d'obtenir, sur une même image, plusieurs couches d'informations complémentaires.

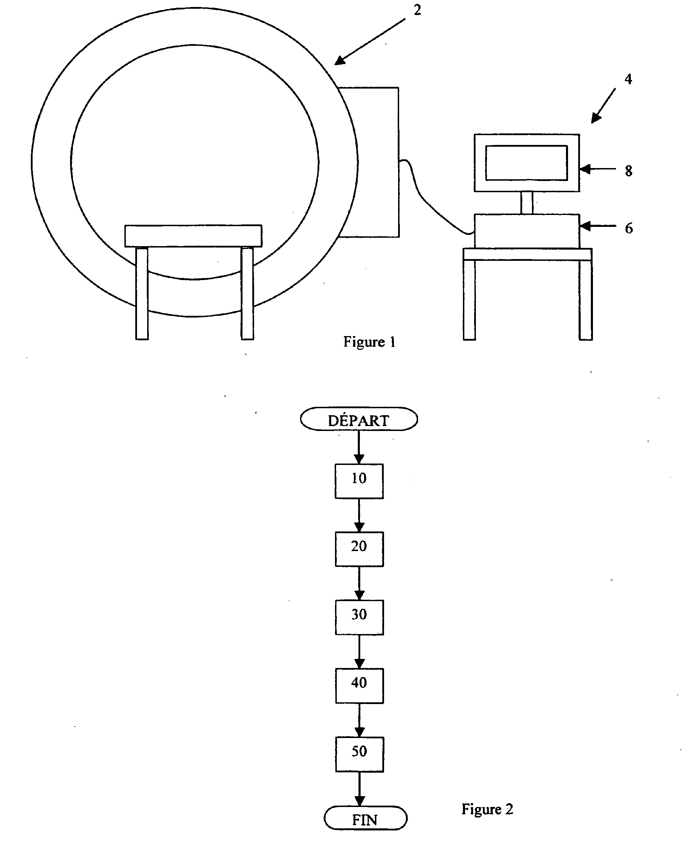

La figure 1 présente un exemple de réalisation d'un dispositif d'estimation du potentiel de croissance selon l'invention. Le dispositif est notamment constitué d'un appareil 2 d'imagerie par résonance magnétique à 1.5 Teslas ou plus, pouvant appliquer des gradients de champs magnétiques selon au moins 6 directions de l'espace.

Le scanner 2 est relié à une station de travail 4 munie d'une unité centrale 6 d'analyse et de traitement d'images. Ainsi, le scanner 2 transmet les images numérisées à la station de travail 4, de sorte que l'unité centrale 6 d'analyse et de traitement d'images mette en oeuvre les étapes (10,20,30,40) successives de la méthode d'estimation du potentiel de croissance de l'infarctus selon l'invention.

De préférence, cette station de travail 4 dispose en outre d'un écran de visualisation 8 permettant au personnel médical d'observer les images numériques obtenues par l'intermédiaire du scanner 2, d'entrer en interaction avec l'unité centrale 6 d'analyse et de traitement des images et éventuellement de visualiser la région correspondant à l'infarctus initial, l'infarctus en croissance et l'infarctus final estimé.

Selon une réalisation particulière, l'écran de visualisation 8 est un écran tactile, ce qui permet de faciliter l'interaction entre le personnel médical et l'écran de visualisation 8.

La figure 2 décrit l'organigramme des étapes (10,20,30,40,50) à suivre pour réaliser une méthode d'estimation du potentiel de croissance de l'infarctus selon l'invention.

La première étape 10 de cette méthode d'estimation du potentiel de croissance consiste à acquérir une séquence d'images numériques par résonance magnétique pondérées en diffusion (ci-après IRM de diffusion) selon un protocole clinique standard bien connu de l'homme du métier. Ce protocole utilise 2 séquences d'images numériques standards appliquées selon des facteur de gradient b également standardisés : $b=0$ $mm^2.s^{-1}$ et $b=1000$ $mm^2.s^{-1}$ dans 6 directions de l'espace. Avantageusement, il est possible d'utiliser des valeurs supplémentaires de gradients ainsi qu'un plus grand nombre de directions d'acquisition, afin d'améliorer la résolution des informations contenues en chaque point de l'espace. Néanmoins, cette augmentation de la résolution se fait au détriment du temps d'acquisition, qui s'en trouve allongé.

Idéalement, l'estimation des risques de propagation de l'infarctus dans les zones de pénombre doit être réalisée dans les six heures suivant l'accident vasculaire cérébral. En effet, cette estimation permet aux cliniciens d'apprécier de façon pertinente le rapport bénéfices/risques lié aux traitements, efficaces mais agressifs, qui doivent être réalisés le plus rapidement possible pour être efficients.

La seule utilisation d'images numériques obtenues par IRM de diffusion présente l'avantage de ne pas nécessiter l'injection intraveineuse de produits de contraste ou le recalage d'images issues de séquences IRM différentes et supplémentaires pour corriger les artefacts dus aux mouvements du patient en cours d'acquisition. Cette acquisition d'images numériques par IRM de diffusion est donc simple et rapide à mettre en oeuvre, ce qui favorise l'intervention d'urgence, primordiale pour permettre un traitement efficace des patients.

Avantageusement, en préalable à l'analyse des images numériques ainsi obtenues, une étape 20 de la méthode d'estimation du potentiel de croissance selon l'invention consiste à recaler et normaliser l'anatomie du sujet dans le référentiel de TALAIRACH. Alternativement, ce recalage peut être effectué dans le référentiel MNI, défini par le *Montreal Neurological Institute*, ou tout autre système de standardisation de l'anatomie du parenchyme cortical. Cette étape 20 permet de repérer la position de n'importe quel point du cerveau d'un individu, en référence à un modèle patron standardisé. L'utilisation d'un tel référentiel facilite également la superposition des cartes issues de bases de données standardisées avec les cartes spécifiques au patient. Ce type de normalisation spatiale peut-être réalisé par un grand nombre de logiciels de neuro-imagerie bien connus de l'homme du métier.

L'étape 30 suivante consiste à localiser et délimiter l'infarctus initial à partir des images numériques résultant de l'IRM de diffusion. Selon un mode de réalisation, cette étape 30 peut-être effectuée par le personnel médical de façon interactive avec l'unité centrale 6 d'analyse et de traitement des images. Il s'agit alors pour l'opérateur de sélectionner sur les différentes images numériques, une région du volume du parenchyme cérébral dont les valeurs de voxels correspondent à un hyper-signal franc dans les séquences de diffusion b1000. Selon des caractéristiques particulières, cette sélection s'effectue par seuillage des images entre deux valeurs de mesures sélectionnées par l'opérateur, qui lui permettent de contourer manuellement la région lésée dans chaque coupe ou bien de cliquer dans le coeur de la lésion pour que le logiciel d'analyse et de traitement d'images sélectionne tous les voxels connexes à cette graine régionale. Ainsi l'opérateur sélectionne spécifiquement l'agrégat de voxels correspondant effectivement à la zone ischémiée initiale dans chaque coupe du volume d'images IRM de diffusion.

Selon un exemple de réalisation alternatif, cette étape 30 est effectuée automatiquement par l'unité centrale 6 d'analyse et de traitement des images. La localisation et la délimitation de l'infarctus initial sont alors effectuées selon un processus automatique de sélection de régions connexes dont les valeurs de voxels en b1000 dépassent significativement une valeur seuil prédéterminée. La valeur seuil dépend de la calibration du scanner IRM 2 en service. Par conséquent, elle doit être déterminée de façon empirique. Selon une réalisation particulière, la valeur seuil peut être issue d'une base d'apprentissage contenant les résultats, recueillis manuellement tel que décrit ci-dessus, d'une trentaine de patients.

Une autre étape 40 consiste à calculer la valeur du coefficient apparent de diffusion (CDA) en chaque point de la séquence d'images de l'encéphale. La valeur du CDA est exprimée en mm$^2$.s$^{-1}$. Ce coefficient est une mesure physique, indépendante du site, du type d'imageur, du champ magnétique de l'imageur et des séquences choisies. Son calcul est tout à fait standard et bien connu de l'homme du métier. Il est effectué à partir des images numériques précitées en gradient b0 et b1000 selon la formule suivante pour chaque voxel:

$$CDA = \frac{- \ln\left(\frac{Sb1000}{Sb0}\right)}{b1000 - b0},$$

où

-     Sbi est la valeur du signal en chaque voxel considéré dans la séquence bi.

Il est à noter que les étapes 30 de localisation et délimitation de l'infarctus initial et 40 de calcul de la valeur du CDA sont indépendantes l'une de l'autre. L'ordre de ces étapes, l'une par rapport à l'autre, peut donc changer sans bouleverser les résultats obtenus par la méthode d'estimation du potentiel de croissance selon l'invention.

Enfin, la méthode selon l'invention comprend une dernière étape 50, au cours de laquelle l'unité centrale 6 d'analyse et de traitement des images modélise la croissance finale de l'infarctus à partir des données recueillies dans les étapes (10,20,30,40) précédentes. Les régions touchées par l'infarctus dans sa phase initiale sont reportées dans l'image du CDA. Ce report se fait de façon automatique et ne nécessite pas de recalage entre les images en b1000 et CDA car elles ont été acquises en même temps. L'ensemble des voxels appartenant à l'infarctus initial servent alors à l'initialisation d'un processus automatique de modélisation de la croissance de l'infarctus.

La modélisation de la croissance de l'infarctus consiste à ajouter récursivement des voxels à la région initialement infarcie, détectée lors de l'étape 30 précédente de la méthode d'estimation selon l'invention. Le modèle sous-jacent de cette modélisation consiste donc à faire grossir virtuellement la lésion dans son état initial en accumulant des voxels des images IRM de diffusion, sous plusieurs conditions :

-     l'intensité des voxels des images IRM de diffusion ne dépasse pas deux valeurs seuils, minimum et maximum, préétablies ;
-     la valeur moyenne du CDA dans la région en croissance reste inférieure à une valeur cible préétablie, par exemple lors d'études rétrospectives sur des groupes de patients ;
-     la surface de la lésion en croissance présente de bonnes propriétés de régularité ;
-     la direction de la croissance suit la direction de gradient minimum du CDA dans l'image lorsque la distribution locale de CDA présente un fort taux d'anisotropie ; et
-     la sélection des voxels par adéquation vis-à-vis d'un atlas probabiliste numérique des régions lésées suite à une occlusion de type équivalent de celle du patient en cours d'étude.

La modélisation de la croissance de l'infarctus est un problème de traitement et d'analyse d'images numériques qui peut être formalisé de multiples façons (modèle d'équilibre énergétique d'un système, processus dynamique modélisé sous la forme d'un jeu d'équations aux dérivées partielles, etc.). Dans tous les cas, et bien que sa transcription algorithmique et logicielle diffère, le modèle de croissance reste le même.

Selon un exemple particulier de réalisation de l'invention, le modèle de croissance est établi dans un formalisme d'équilibre énergétique. La lésion dans son état final est alors modélisée selon plusieurs paramètres d'énergie élémentaires Ei dont la combinaison linéaire définit un indice d'énergie globale E. Par construction, l'indice d'énergie globale E est minimum lorsque la lésion a atteint son état de croissance final.

Les paramètres d'énergie élémentaires définissent respectivement :

- un contrôle de l'écart de la moyenne du CDA au sein de la zone en croissance ($E_R$). En effet, les modèles physiopathologiques prédisent que la moyenne régionale du CDA décroît faiblement mais significativement au sein de la zone à risque d'infarcissement futur. Selon une réalisation particulière, la valeur cible pré-établie vers laquelle tend la valeur régionale moyenne du CDA à l'intérieur de la région en croissance est sensiblement égale à 740 mm$^2$.s$^{-1}$. Alternativement, cette valeur cible est exprimée en rapport avec une région de contrôle prise dans le parenchyme controlatéral à la lésion, et vaut, par exemple, 0.93 fois la valeur moyenne du CDA dans cette région de contrôle.

- un contrôle de la valeur du CDA en chaque voxel candidat à l'ajout au volume en croissance de l'infarctus afin qu'il reste dans des valeurs admissibles, prédéfinies sur une base d'apprentissage ($E_V$). Ainsi seuls les voxels modélisant le tissu cérébral et non le liquide céphalo-rachidien sont pris en compte dans le calcul de cette équation fonctionnelle d'énergie;

- un contrôle de la régularité de la forme de la surface externe de l'infarctus en croissance $E_S$.

- un contrôle de la direction de la croissance afin qu'elle se déroule selon le gradient minimum de variation spatiale de CDA $E_{AN}$ ;

- un contrôle de l'adéquation de la croissance avec un atlas probabiliste d'atteinte de régions cérébrales par un infarctus ischémique $E_P$.

Ces paramètres d'énergie élémentaire $E_I$ sont combinés selon la formule suivante, définissant l'indice d'énergie globale E :

$$E = \sum_{v \in INF} (1 - FA(v)) \cdot E_{IS}(v) - \gamma \cdot FA(v) \cdot E_{AN}(v) + \theta \cdot Ep(v),$$

où

-    FA est le coefficient d'anisotropie fractionnelle moyen, dont la définition est connue de l'homme du métier, caractérisant la répartition du CDA aux alentours des voxels à la surface de la lésion en cours de croissance ;
-    $\theta$ et $\gamma$ sont des paramètres scalaires fixes.
-    $E_{AN}$ est l'indice de corrélation cumulée entre le flot de vecteurs gradients définis en chaque voxel de la carte de CDA $\overline{V}_{CDA}$, bien connu de l'homme du métier, et les gradients calculés en tout point du masque binaire de la lésion en croissance $\overline{V}_{INF}$ :

$$E_{AN} = \frac{1}{\left\|\vec{V}_{CDA}\right\|\left\|\vec{V}_{INF}\right\|} \sum_{v \in INF} \vec{V}_{CDA}(v) \cdot \vec{V}_{INF}(v)$$

-    $E_P$ est un facteur d'adéquation établi entre un atlas probabiliste et la croissance en cours de la lésion. La probabilité $p(v)$ que le voxel v appartiennent à l'infarctus dans sa forme finale est obtenue de façon empirique, sur des patients ayant souffert d'un type d'infarctus similaire, ayant pour origine la même occlusion initiale. Le facteur d'adéquation Ep cumule les probabilités d'atteinte de chaque voxel appartenant à la lésion en cours de croissance, telle qu'estimée à chaque itération :

$$E_p = \sum_{v \in INF} p(v) \; ;$$

où

- $E_{IS}$ est une combinaison linéaire de trois paramètres d'énergie élémentaires:

$$E_{IS} = E_R + \delta_V E_V + \beta_S E_S,$$

avec

$$E_R = \left(\frac{i_{INF} - \bar{i}_{INF}}{\sigma_{INF}}\right)^2, \quad E_V = \left(\frac{i(v) - \bar{i}_{IG}}{\sigma_{IG}}\right)^2, \quad E_S = \left(\frac{N_v - N/2}{\delta}\right)^\beta$$

- $i_{NF}$ (resp. $\sigma_{INF}$) est la moyenne empirique (resp. l'écart type) de l'intensité du CDA dans la région en croissance *INF* ;
- $\bar{i}_{INF}$ est une valeur cible de la moyenne régionale coeur de la zone de pénombre ischémique ;
- $i(v)$ est la valeur du CDA au voxel *v*;
- $\bar{i}_{IG}$ (resp. $\sigma_{IG}$) est un *a priori* concernant les valeurs de CDA moyennes (resp. en écart type) aux voxels dans la région de croissance *IG* ;
- $N_{INF}$ (resp. $N_{IG}$) le nombre de voxels dans les régions *INF* et *IG*, respectivement ;
- $E_S$ est le potentiel de régularisation selon Ising, bien connu de la communauté scientifique spécialiste en traitement des images numériques ;
- $N$ est le nombre total de voxels voisins à un voxel candidat à l'inclusion dans la région *INF* (26 par exemple, en 3 dimensions), dont $N_v$ appartiennent déjà *INF.*
- $\delta$ et $\beta$ sont des paramètres scalaires fixes ;

La modélisation de la croissance est menée de manière itérative par accumulation successive des voxels immédiatement voisins de la région en croissance. L'accumulation s'achève lorsque l'indice d'énergie globale E de la lésion virtuelle est minimisé. Les voxels sélectionnés constituent dès lors la région de l'infarctus final estimé.

Selon un exemple de réalisation alternatif, il est également possible de considérer un modèle de croissance qui emploie un formalisme dynamique variationnel de type « ligne de niveau » ou level-set, bien connu de l'homme du métier, notamment par résolution d'équations différentielles portant sur des critères identiques de sélection des voxels. L'utilisation d'un tel formalisme permettrait d'aboutir exactement aux mêmes résultats en termes de qualité de modélisation et d'anticipation de la croissance finale de la lésion. Seules des différences de performances en temps de calcul pourraient distinguer ces deux modes de réalisation alternatifs.

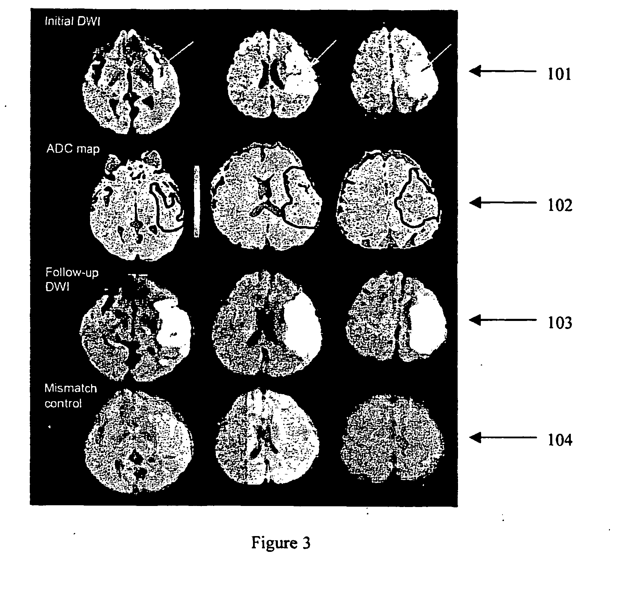

Enfin, la figure 3 présente plusieurs séquences d'images 101,102,103,104 représentant les croissances réelles et prédites de l'infarctus cérébral chez un patient. Selon un mode de réalisation, ces images sont visualisées par le personnel médical sur l'écran de visualisation 8.

La première séquence d'images 101 représente 3 coupes illustratives du parenchyme cortical, obtenues lors de l'étape initiale 10 d'acquisition d'images IRM de diffusion. L'infarctus déjà formé apparaît sur cette première séquence d'images en hypersignal franc blanc.

La seconde séquence d'images 102 représente la carte du coefficient apparent de diffusion encodé en fausses couleurs. La prédiction de croissance obtenue par la méthode d'estimation selon l'invention est incorporée sur cette carte et prédit une taille de l'infarctus final contourée en noir.

La troisième séquence d'images 103 représente 3 coupes illustratives obtenues par IRM de diffusion 24 heures après l'accident vasculaire cérébral. La phase de croissance de l'infarctus est alors achevée et la taille finale effective de cet infarctus est facilement identifiable de la même façon que dans la première séquence d'images, en hypersignal blanc.

Enfin, la quatrième séquence d'images 104 permet d'obtenir par un contrôle visuel l'adéquation entre la taille de l'infarctus final, en hypersignal blanc comme dans la troisième séquence d'images, et la prédiction automatique obtenue par la méthode d'estimation selon l'invention, représentée en bleu.

Bien entendu, l'invention n'est pas limitée aux exemples de réalisation décrits et représentés ci-dessus. Il est entendu que l'homme du métier est à même de réaliser des variantes de l'invention sans pour autant sortir du cadre de ce brevet.

## Revendications

1. Méthode d'estimation du potentiel de croissance des infarctus cérébraux, la méthode comportant :

   - une étape (10) d'acquisition de séquences d'images IRM de diffusion ;
   - une étape (40) de calcul du coefficient apparent de diffusion (CDA) en une multitude de points ou voxels du parenchyme cortical ;
   - une étape (30) de localisation et délimitation de l'infarctus initial ;

   **caractérisée en ce que** la méthode d'estimation comporte en outre une étape (50) de modélisation de l'évolution de l'infarctus à partir d'un modèle de croissance établie par la minimisation itérative d'un indice d'énergie globale E défini par une combinaison linéaire de paramètres d'énergie élémentaires dépendant de l'intensité du CDA.

2. Méthode d'estimation du potentiel de croissance des infarctus cérébraux selon la revendication 1, dans laquelle la combinaison linéaire comporte un paramètre d'énergie élémentaire $E_R$ selon lequel la valeur régionale moyenne du CDA à l'intérieur de la région en croissance tend vers une valeur cible pré-établie.

3. Méthode d'estimation du potentiel de croissance des infarctus cérébraux selon la revendication 2, dans laquelle le paramètre d'énergie élémentaire $E_R$ se traduit par la fonction mathématique suivante :

$$E_R = \left( \frac{i_{INF} - \bar{i}_{INF}}{\sigma_{INF}} \right)^2 ,$$

où

   - $i_{INF}$ (resp. $\sigma_{INF}$) est la moyenne empirique (resp. l'écart type) de l'intensité du CDA dans la région en croissance $INF$ ;
   - $\bar{i}_{INF}$ est une valeur cible de la moyenne régionale coeur de la zone de pénombre ischémique.

4. Méthode d'estimation du potentiel de croissance des infarctus cérébraux selon la revendication, dans laquelle la valeur cible pré-établie vers laquelle tend la valeur régionale moyenne du CDA à l'intérieur de la région en croissance est sensiblement égal à 740 mm$^2$.s$^{-1}$.

5. Méthode d'estimation du potentiel de croissance des infarctus cérébraux selon l'une des revendications 1 à 3, dans laquelle la valeur cible pré-établie vers laquelle tend la valeur régionale moyenne du CDA à l'intérieur de la région en croissance est sensiblement égal à 0.93 fois la valeur moyenne régionale du CDA dans une région saine controlatérale.

6. Méthode d'estimation du potentiel de croissance des infarctus cérébraux selon l'une au moins des revendications précédentes, dans laquelle la combinaison linéaire comporte un paramètre d'énergie élémentaire $E_S$ selon lequel l'enveloppe de l'infarctus possède une surface régulière.

7. Méthode d'estimation du potentiel de croissance des infarctus cérébraux selon la revendication 6, dans laquelle le paramètre d'énergie élémentaire Es se traduit par la fonction mathématique suivante :

$$E_S = \sum_{i \in INF} \left( \frac{N_v - N/2}{\delta} \right)^\beta ,$$

où

   - $N_{INF}$ (resp. $N_{IG}$) est le nombre de voxels dans les régions $INF$ et IG, respectivement ;
   - $E_S$ est potentiel de régularisation selon Ising, bien connu de la communauté en traitement numérique des images ;
- N est le nombre total de voxels voisins à un voxel candidat à l'inclusion dans la région *INF* (26 par exemple, en 3 dimensions), dont $N_v$ appartiennent déjà *INF* ;
- δ et β sont des paramètres scalaires fixes.

8.  Méthode d'estimation du potentiel de croissance des infarctus cérébraux selon l'une au moins des revendications précédentes, dans laquelle la combinaison linéaire comporte un paramètre d'énergie élémentaire $E_V$ selon lequel seuls les voxels modélisant le tissu cérébral sont pris en compte dans le calcul de cette indice d'énergie globale E.

9.  Méthode d'estimation du potentiel de croissance des infarctus cérébraux selon la revendication 8, **caractérisé en ce que** le paramètre d'énergie élémentaire $E_v$ se traduit par la fonction mathématique suivante :

$$E_V = \sum_{v \in IG} \left( \frac{i(v) - \bar{i}_{IG}}{\sigma_{IG}} \right)^2 ,$$

où

- $i(v)$ est la valeur du CDA au voxel v ;
- $\bar{i}_{IG}$ (resp. $\sigma_{IG}$) est un a priori concernant les valeurs de CDA moyennes (resp. en écart type) aux voxels dans la région de croissance *IG* ;
- $N_{INF}$ (resp. $N_{IG}$) le nombre de voxels dans les régions *INF* et *IG*, respectivement.

10. Méthode d'estimation du potentiel de croissance des infarctus cérébraux selon l'une des revendications précédentes, dans laquelle la combinaison linéaire comporte un paramètre d'énergie élémentaire $E_P$ définissant les probabilités de croissance de l'infarctus à chaque voxel à partir de schémas empiriques.

11. Méthode d'estimation du potentiel de croissance des infarctus cérébraux selon la revendication 10, **caractérisé en ce que** le paramètre d'énergie élémentaire $E_P$ se traduit par la fonction mathématique suivante :

$$E_p = \sum_{v \in INF} p(v) ,$$

où

- p(v) est la probabilité a priori que le voxel v appartienne à l'infarctus dans sa forme finale.

12. Méthode d'estimation du potentiel de croissance des infarctus cérébraux selon l'une au moins des revendication précédentes, dans laquelle la combinaison linéaire comporte un paramètre d'énergie élémentaire $E_{AN}$ définissant un modèle de croissance anisotrope, réalisé par le calcul du gradient local du coefficient de diffusion apparent.

13. Méthode d'estimation du potentiel de croissance des infarctus cérébraux selon la revendication 12, **caractérisé en ce que** le paramètre d'énergie élémentaire $E_{AN}$ se traduit par la fonction mathématique suivante :

$$E_{AN} = \frac{1}{\left\| \vec{V}_{CDA} \right\| \left\| \vec{V}_{INF} \right\|} \sum_{v \in INF} \vec{V}_{CDA}(v) \cdot \vec{V}_{INF}(v) ,$$

où

- $\overline{V}_{CDA}$ est le flot de vecteurs gradients défini en chaque voxel de la carte de CDA ; et
- $\overline{V}_{INF}$ les gradients calculés en tout point du masque binaire de la lésion en croissance.

**14.** Méthode d'estimation du potentiel de croissance des infarctus cérébraux selon l'une au moins des revendications précédentes, dans laquelle l'étape (30) de localisation et de délimitation de l'infarctus initial est automatique.

**15.** Dispositif d'estimation du potentiel de croissance des infarctus cérébraux mettant en oeuvre une méthode selon l'une des revendications précédentes.

**Claims**

**1.** Method for estimating the growth potential of cerebral infarcts, the method comprising:

> - a step (10) of acquiring diffusion MRI image sequences;
> - a step (40) of calculating the apparent diffusion coefficient (ADC) at a multitude of points or voxels of the cortical parenchyma;
> - a step (30) of locating and delimiting the initial infarct;

**characterized in that** the method of estimation furthermore comprises a step (50) of modelling the evolution of the infarct on the basis of a model of growth established by the iterative minimization of a global energy index E defined by a linear combination of elementary energy parameters dependent on the intensity of the ADC.

**2.** Method for estimating the growth potential of cerebral infarcts according to claim 1, in which the linear combination comprises an elementary energy parameter $E_R$ according to which the mean regional value of the ADC inside the growth region tends towards a pre-established target value.

**3.** Method for estimating the growth potential of cerebral infarcts according to claim 2, in which the elementary energy parameter $E_R$ is conveyed by the following mathematical function:

$$E_R = \left( \frac{i_{INF} - \bar{i}_{INF}}{\sigma_{INF}} \right)^2,$$

where

> - $i_{INF}$ (resp. $\sigma_{INF}$) is the empirical mean (resp. the standard deviation) of the intensity of the ADC in the growth region *INF*;
> - $\bar{i}_{INF}$ is a target value of the core regional mean of the ischemic penumbra zone.

**4.** Method for estimating the growth potential of cerebral infarcts according to the claims, in which the pre-established target value towards which the mean regional value of the ADC inside the growth region tends is substantially equal to 740 mm$^2$.s$^{-1}$.

**5.** Method for estimating the growth potential of cerebral infarcts according to one of claims 1 to 3, in which the pre-established target value towards which the mean regional value of the ADC inside the growth region tends is substantially equal to 0.93 times the regional mean value of the ADC in a contralateral healthy region.

**6.** Method for estimating the growth potential of cerebral infarcts according to at least one of the preceding claims, in which the linear combination comprises an elementary energy parameter $E_S$ according to which the envelope of the infarct possesses a regular surface.

**7.** Method for estimating the growth potential of cerebral infarcts according to claim 6, in which the elementary energy parameter $E_S$ is conveyed by the following mathematical function:

$$E_S = \sum_{v \in INF} \left( \frac{N_v - N/2}{\delta} \right)^{\beta}$$

where

- $N_{INF}$ (resp. $N_{IG}$) is the number of voxels in the regions *INF* and *IG*, respectively;
- $E_S$ is regularization potential according to Ising, well known to the digital image processing community;
- $N$ is the total number of voxels neighbouring a candidate voxel for inclusion in the region *INF* (26 for example, in 3 dimensions), $N_v$ of which already belong to *INF;*
- $\delta$ and $\beta$ are fixed scalar parameters.

8. Method for estimating the growth potential of cerebral infarcts according to at least one of the preceding claims, in which the linear combination comprises an elementary energy parameter $E_V$ according to which only the voxels modelling the cerebral tissue are taken into account in calculating this global energy index E.

9. Method for estimating the growth potential of cerebral infarcts according to claim 8, **characterized in that** the elementary energy parameter $E_v$ is conveyed by the following mathematical function:

$$E_V = \sum_{v \in IG} \left( \frac{i(v) - \bar{i}_{IG}}{\sigma_{IG}} \right)^2,$$

where

- *i(v)* is the value of the ADC at the voxel *v;*
- $\bar{i}_{IG}$ (resp. $\sigma_{IG}$) is an a priori relating to the mean (resp. standard deviation) values of ADC at the voxels in the growth region *IG*;
- $N_{INF}$ (resp. $N_{IG}$) the number of voxels in the regions *INF* and IG, respectively.

10. Method for estimating the growth potential of cerebral infarcts according to one of the preceding claims, in which the linear combination comprises an elementary energy parameter $E_p$ defining the probabilities of growth of the infarct at each voxel on the basis of empirical schemes.

11. Method for estimating the growth potential of cerebral infarcts according to claim 10, **characterized in that** the elementary energy parameter $E_p$ is conveyed by the following mathematical function:

$$E_p = \sum_{v \in INF} p(v),$$

where

- p(v) is the a priori probability that the voxel v belongs to the infarct in its final form.

12. Method for estimating the growth potential of cerebral infarcts according to at least one of the preceding claims, in which the linear combination comprises an elementary energy parameter $E_{AN}$ defining an anisotropic growth model, effected by calculating the local gradient of the apparent diffusion coefficient.

13. Method for estimating the growth potential of cerebral infarcts according to claim 12, **characterized in that** the elementary energy parameter $E_{AN}$ is conveyed by the following mathematical function:

$$E_{AN} = \frac{1}{\|\vec{V}_{CDA}\|\|\vec{V}_{INF}\|} \sum_{v \in INF} \vec{V}_{CDA}(v) \cdot \vec{V}_{INF}(v),$$

where

- $\overline{V}_{CDA}$ is the gradient vector flow defined at each voxel of the ADC map; and
- $\overline{V}_{INF}$ the gradients calculated at any point of the binary mask of the lesion under growth.

**14.** Method for estimating the growth potential of cerebral infarcts according to at least one of the preceding claims, in which method the step (30) of locating and delimiting the initial infarct is automatic.

**15.** Device for estimating the growth potential of cerebral infarcts implementing a method according to one of the preceding claims.

**Patentansprüche**

**1.** Verfahren zum Abschätzen des Wachstumspotentials der Gehirninfarkte, wobei das Verfahren Folgendes umfasst:

- einen Schritt (10) zum Erfassen von IRM-Ausbreitungsbildsequenzen;
- einen Schritt (40) zum Berechnen des scheinbaren Ausbreitungskoeffizienten (CDA) an einer Vielzahl von Punkten oder Voxeln des kortikalen Parenchyms;
- einen Schritt (30) zum Lokalisieren und Abgrenzen des anfänglichen Infarkts;
**dadurch gekennzeichnet, dass** das Abschätzungsverfahren außerdem einen Schritt (50) zum Modellieren der Entwicklung des Infarkts anhand eines Wachstumsmodells umfasst, das durch die iterative Minimierung eines globalen Energieindex E erstellt wird, der für eine lineare Kombination von elementaren Energieparametern in Abhängigkeit von der Intensität des CDA definiert ist.

**2.** Verfahren zum Abschätzen des Wachstumspotentials der Gehirninfarkte nach Anspruch 1, bei dem die lineare Kombination einen elementaren Energieparameter $E_R$ umfasst, gemäß dem der regionale Mittelwert des CDA im Inneren des Wachstumsbereichs gegen einen vorher festgelegten Zielwert strebt.

**3.** Verfahren zum Abschätzen des Wachstumspotentials der Gehirninfarkte nach Anspruch 2, bei dem der elementare Energieparameter $E_R$ in der folgenden mathematischen Funktion zum Ausdruck kommt:

$$E_R = \left( \frac{i_{INF} - \bar{i}_{INF}}{\sigma_{INF}} \right)^2,$$

wobei

- $i_{INF}$ (bzw. $\sigma_{INF}$) der empirische Mittelwert (bzw. die Standardabweichung) der Intensität des CDA im Wachstumsbereich *INF* ist;
- $\bar{i}_{INF}$ ein Zielwert des regionalen Kernmittelwerts der ischämischen Halbschattenzone ist.

**4.** Verfahren zum Abschätzen des Wachstumspotentials der Gehirninfarkte nach Anspruch, bei dem der vorher festgelegte Zielwert, gegen den der regionale Mittelwert des CDA im Inneren des Wachstumsbereichs strebt, im Wesentlichen gleich 740 mm$^2$s$^{-1}$ ist.

**5.** Verfahren zum Abschätzen des Wachstumspotentials der Gehirninfarkte nach einem der Ansprüche 1 bis 3, bei dem der vorher festgelegte Zielwert, gegen den der regionale Mittelwert des CDA im Inneren des Wachstumsbereichs strebt, im Wesentlichen gleich 0,93-mal der regionale Mittelwert des CDA in einem kontralateralen gesunden Bereich ist.

6. Verfahren zum Abschätzen des Wachstumspotentials der Gehirninfarkte nach mindestens einem der vorangehenden Ansprüche, bei dem die lineare Kombination einen elementaren Energieparameter $E_S$ umfasst, gemäß dem die Hülle des Infarkts eine regelmäßige Oberfläche besitzt.

7. Verfahren zum Abschätzen des Wachstumspotentials der Gehirninfarkte nach Anspruch 6, bei dem der elementare Energieparameter Es in der folgenden mathematischen Funktion zum Ausdruck kommt:

$$E_S = \sum_{v \in INF} \left( \frac{N_v - N/2}{\delta} \right)^\beta,$$

wobei

- $N_{INF}$ (bzw. $N_{IG}$) die Anzahl von Voxeln in den Bereichen *INF* bzw. *IG* ist;
- $E_S$ das Regulierungspotential nach Ising ist, das von der Gemeinschaft zur digitalen Bildverarbeitung gut bekannt ist;
- N die Gesamtzahl von zu einem Kandidatenvoxel benachbarten Voxeln im Einschluss im Bereich *INF* ist (beispielsweise 26, in 3 Dimensionen), wovon $N_V$ bereits zu *INF* gehört;
- $\delta$ und $\beta$ feste skalare Parameter sind.

8. Verfahren zum Abschätzen des Wachstumspotentials der Gehirninfarkte nach mindestens einem der vorangehenden Ansprüche, bei dem die lineare Kombination einen elementaren Energieparameter $E_V$ umfasst, gemäß dem nur die Voxel, die das Gehirngewebe modellieren, in der Berechnung dieses globalen Energieindex E berücksichtigt werden.

9. Verfahren zum Abschätzen des Wachstumspotentials der Gehirninfarkte nach Anspruch 8, **dadurch gekennzeichnet, dass** der elementare Energieparameter $E_V$ in der folgenden mathematischen Funktion zum Ausdruck kommt:

$$E_V = \sum_{v \in IG} \left( \frac{i(v) - \bar{i}_{IG}}{\sigma_{IG}} \right)^2,$$

wobei

- $i(v)$ der Wert des CDA am Voxel v ist;
- $\bar{i}_{IC}$ (bzw. $\sigma_{IG}$) ein a priori in Bezug auf die CDA-Mittelwerte (bzw. die Standardabweichung) für die Voxel im Wachstumsbereich IG ist;
- $N_{INF}$ (bzw. $N_{IG}$) die Anzahl von Voxeln in den Bereichen *INF* bzw. *IG* ist.

10. Verfahren zum Abschätzen des Wachstumspotentials der Gehirninfarkte nach einem der vorangehenden Ansprüche, bei dem die lineare Kombination einen elementaren Energieparameter $E_P$ umfasst, der die Wachstumswahrscheinlichkeiten des Infarkts an jedem Voxel anhand von empirischen Schemen definiert.

11. Verfahren zum Abschätzen des Wachstumspotentials der Gehirninfarkte nach Anspruch 10, **dadurch gekennzeichnet, dass** der elementare Energieparameter $E_P$ in der folgenden mathematischen Funktion zum Ausdruck kommt:

$$E_p = \sum_{v \in INF} p(v),$$

wobei

     - p(v) die Wahrscheinlichkeit a priori ist, dass das Voxel v zum Infarkt in seiner Endform gehört.

**12.** Verfahren zum Abschätzen des Wachstumspotentials der Gehirninfarkte nach mindestens einem der vorangehenden Ansprüche, bei dem die lineare Kombination einen elementaren Energieparameter $E_{AN}$ umfasst, der ein anisotropes Wachstumsmodell definiert, das durch die Berechnung des lokalen Gradienten des scheinbaren Ausbreitungskoeffizienten verwirklicht wird.

**13.** Verfahren zum Abschätzen des Wachstumspotentials der Gehirninfarkte nach Anspruch 12, **dadurch gekennzeichnet, dass** der elementare Energieparameter $E_{AN}$ in der folgenden mathematischen Funktion zum Ausdruck kommt:

$$E_{AN} = \frac{1}{\left\| \vec{V}_{CDA} \right\| \left\| \vec{V}_{INF} \right\|} \sum_{v \in INF} \vec{V}_{CDA}(v) \cdot \vec{V}_{INF}(v),$$

wobei

     - $\overline{V}_{CDA}$ die Menge von Gradientenvektoren ist, die an jedem Voxel der CDA-Karte definiert ist; und
     - $\overline{V}_{INF}$ die an jedem Punkt der binären Maske der wachsenden Läsion berechneten Gradienten sind.

**14.** Verfahren zum Abschätzen des Wachstumspotentials der Gehirninfarkte nach mindestens einem der vorangehenden Ansprüche, bei dem der Schritt (30) der Lokalisierung und der Abgrenzung des anfänglichen Infarkts automatisch ist.

**15.** Vorrichtung zum Abschätzen des Wachstumspotentials der Gehirninfarkte, die ein Verfahren nach einem der vorangehenden Ansprüche einsetzt.

Figure 1

Figure 2

Figure 3

**EP 2 036 044 B1**

**RÉFÉRENCES CITÉES DANS LA DESCRIPTION**

**Documents brevets cités dans la description**

- WO 07058632 A
- WO 02069799 A